# FASCICULE DE BREVET EUROPEEN

(11) **EP 0 576 317 B1**
(45) Date de publication et mention de la délivrance du brevet: **29.08.2001**
(21) Numéro de dépôt: 93401436.6
(22) Date de dépôt: 04.06.1993
(51) Int. Cl.: A61F 2/34, A61F 2/38

(54) **Dispositif prothétique articulaire**
Prothetische Gelenkvorrichtung
Articular prosthetic device

(30) Priorité: 10.06.1992 FR 9206955
(43) Date de publication de la demande: 29.12.1993
(73) Titulaire: Deckner, André Georges, F-75015 Paris (FR)
(72) Inventeur: Deckner, André Georges, F-75015 Paris (FR)

(56) Documents cités:
- EP-A- 0 119 321
- EP-A- 0 144 588
- EP-A- 0 253 941
- EP-A- 0 313 762
- DE-A- 3 840 468
- FR-A- 2 637 177
- FR-A- 2 645 433

## Description

L'invention est du domaine médical et concerne plus particulièrement les dispositifs prothétiques articulaires à coque pour articulations humaines et animales qui affectent la conformation de type à surface conique telle qu'à surface sphérique ou énarthrose, à surface ellipsoïdale ou condylarthrose ou enfin à surface curviligne dite en selle ou pédarthrose.

Le sens de surface conique est pris ici au sens large en tant qu'affectant la configuration d'un cône c'est-à-dire d'une surface engendrée par une droite mobile (génératrice) qui passe par un point fixe (sommet) en s'appuyant sur une courbe (directrice).

D'une manière générale, toute prothèse articulaire vise à relier fonctionnellement un organe défaillant qui peut être appelé os porteur à un autre organe défaillant qui est l'os porté.

On rencontre fréquemment ce genre de prothèse sur la hanche humaine ce qui pose de difficiles problèmes mécaniques à résoudre en raison du fait qu'elle présente le plus grand cône d'amplitude. La charge la plus élevée dépasse quelquefois 100 kg avec pilonnage important dû à la marche. Le système est destiné à fonctionner 10 à 20 ans et doit subir plus d'un million de pas par an.

Lors d'une intervention de ce genre, on met en place, côté os portant, une tête fémorale fixée sur le fémur et, côté os porté, un implant cotyloïdien.

L'intervention peut porter sur l'un ou l'autre de ces éléments ou les deux.

Habituellement, les liaisons de prothèses articulaires faisaient appel à des emboîtements lâches entre les éléments avec inserts de type conique ou sphérique ou autre qui ne donnaient pas satisfaction en raison du fait que des jeux résiduels apparaissaient toujours à l'usage.

On connaît, par le brevet EP 0253941, un emboîtement qui n'est pas conique et dans lequel les parties de tête et de prothèse sont cylindriques et les degrés de liberté sont annulés par appui sommital et assujettissement d'un écrou fournissant un plan radial d'appui. En outre, des renflements annulaires permettent un appui aléatoire d'accroissement de surface de contact entre un berceau et la tête de prothèse. Cet appui aléatoire n'apparaît que lorsque la tête s'est partiellement enfoncée sous les efforts et ce téton s'est déformé élastiquement.

On connaît également, par le brevet EP 0144588, un mode d'assemblage monocoque dans lequel l'emboîtement n'est pas positif par le fait de fentes d'élasticité devant assurer un parfait logement du corps du cotyle dans l'anneau extérieur. Lorsque cet anneau extérieur se déforme radialement sous une pression axiale, les arcs entre fentes ont conservé leurs rayons d'origine alors que le corps du cotyle reste indéformable par construction ce qui fait qu'il ne porte plus alors que sur les extrémités de ces arcs et qu'il n'y a plus alors congruence entre les rayons de contact du corps de cotyle et l'anneau extérieur. Ce brevet ne comporte pas de cônes multiples et préconise une intervention n'enlevant pas le minimum de matière vivante (0S).

Ces différents brevets n'entrent pas dans le champ d'application de l'invention.

D'autres brevets, tel le brevet EP 0253941 qui présente un système flottant dit à amortissement, le brevet EP 0313762 qui réalise un blocage d'un élément intermédiaire sur un élément fixe par un simple encliquetage, le brevet FR 2637177 qui préconise un striage d'ancrage, le brevet FR 2645433 qui montre un mode d'assemblage dans lequel trois cupules sont à emboîtement sphérique avec des zônes de picots, le brevet EP 119321 qui énonce un mode de liaison avec anneaux et le brevet DE 3840468 qui préconise la mise en place d'un élément rigide à l'intérieur d'un élément souple.

La surface intérieure de l'élément extérieur souple n'a pas de forme géométrique revendiquée et la surface extérieure de l'élément intérieur comporte une forme-enveloppe d'allure générale conique, mais sa macrostructure de surface est constituée par un filetage continu de section locale triangulaire.

Ces différents brevets n'entrent pas non plus dans le domaine de l'invention.

Il est à noter que, si une prothèse de hanche est de réalisation difficile mécaniquement, la conformation d'une autre articulation telle que celle du genou du type pédarthrose est des plus complexes à résoudre au plan géométrique en plus des contraintes sus-mentionnées.

L'invention propose un dispositif prothétique à emboîtement par liaison à gradins coniques entre, d'une part un insert et une coque, et d'autre part entre ce même insert et un siège recevant la tête rotulante de prothèse ; lesdits emboîtements qui n'ont pas les inconvénients antérieurs, assurent des assemblages autobloqués sans jeu étant noté que ledit insert et ledit siège peuvent être d'une seule pièce ce qui a l'avantage de supprimer l'un de ces deux emboîtements.

La coque est fixée à l'os porté par tout moyen approprié connu, tels que : vissage, cimentage, etc ...

L'invention sera bien comprise dans la suite du texte à l'appui des dessins annexés.
Sur les dessins :
- la figure 1 est une vue très schématique en coupe arrachée montrant l'ensemble du dispositif prothétique selon l'invention,
- la figure 2 est une vue schématique en coupe d'une prothèse de hanche
- la figure 3 est une vue en perspective d'une articulation de genou tracée par maillage.

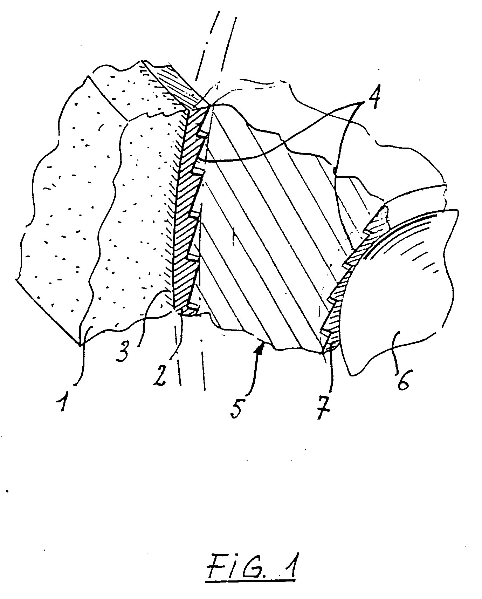

Sur la figure 1 qui représente en vue arrachée un dispositif prothétique théorique, on voit l'os porté 1 recevant la coque 2 par un moyen de fixation approprié 3 ; ladite coque 2 recevant elle-même par un emboîtement de liaison 4 un insert repéré 5 dans son ensemble, tandis que ledit insert permet à la tête rotulante 6 de s'articuler, qu'il y ait ou non un siège d'interposition 7.

Bien entendu, l'usinage de l'os 1 est connu classiquement ainsi que le moyen de fixation 3 qui peut s'effectuer par cimentage, vissage par pas de vis ou par vis multiples de même que la tête de prothèse, qui peut affecter toute dimension en fonction du matériau constitutif.

La fixation de la tête de prothèse 6 à l'os portant est classique et n'est pas représentée.

D'une manière générale et non exclusive, la coque 2 peut être en titane avec une épaisseur compatible avec une certaine souplesse recherchée, l'insert 5 est massivement en une matière telle que du polyéthylène et le siège est de nature compatible avec la tête afin d'offrir un glissement optimum et une usure minimale.

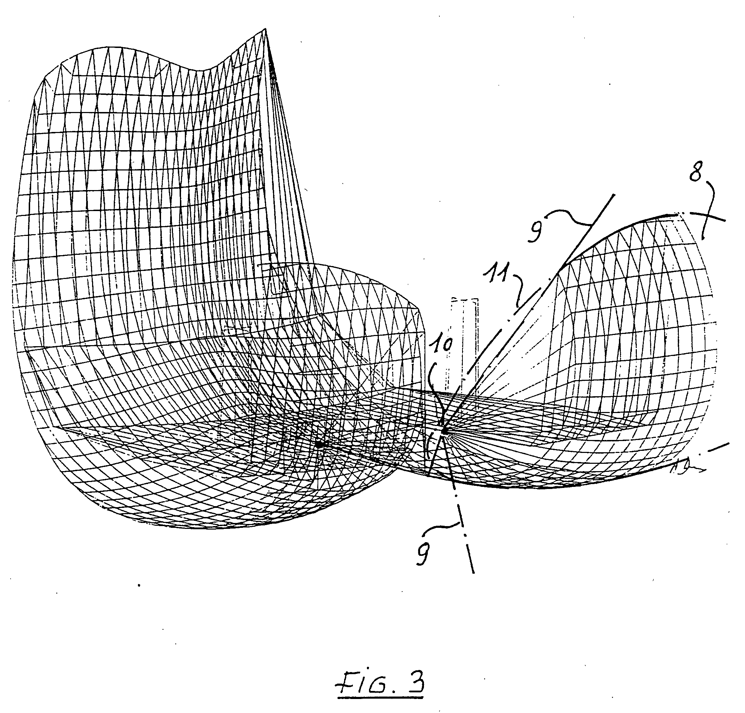

La figure 3 montre la complexité géométrique d'une prothèse de genou ou de type pédarthrose sur laquelle on voit que, comme mentionné précédemment, la surface 8 est une approximation d'un élément de surface conique et les surfaces 9 et 10, une autre approximation des deux autres surfaces coniques ; l'invention pouvant entrer dans le champ d'application d'une telle articulation sans perdre de vue que les surfaces d'articulation de ce type sont gauches et de grande complexité.

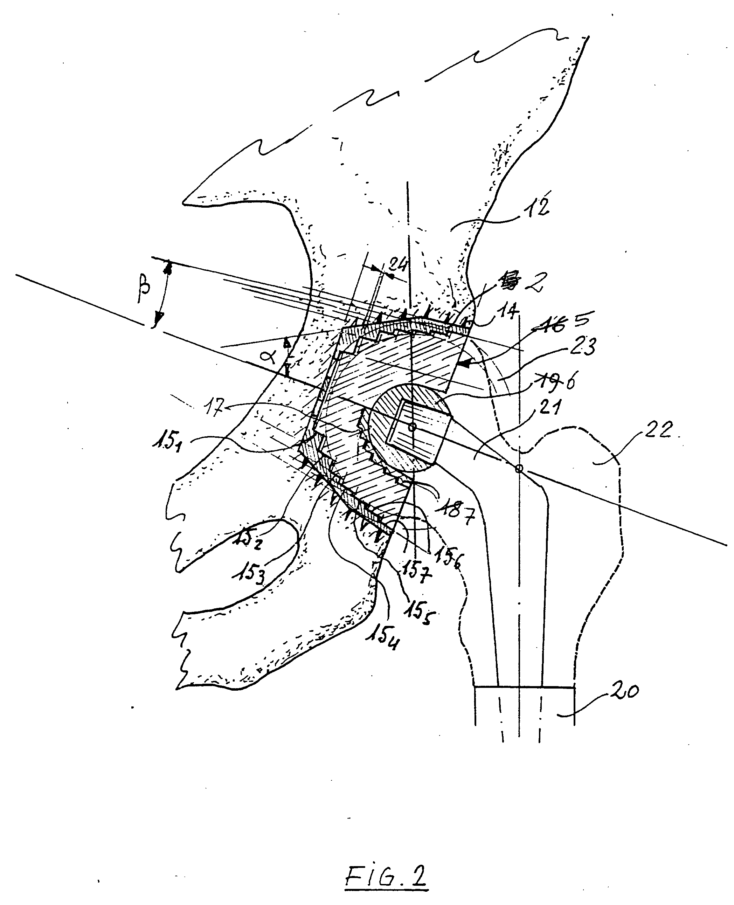

Sur cette figure 2, on voit là encore, l'os porté 1 recevant la coque 2 par un moyen de fixation qui est dans ce cas un filet de vissage 14 et ladite coque 2 reçoit par emboîtement avec liaison à gradins coniques repérée 15 dans son ensemble, un insert repéré 5 dans son ensemble et qui peut recevoir, également par liaison à gradins coniques repérée 17 dans son ensemble, un siège 7.

L'insert articule la tête fémorale 6 fixée sur l'os portant 20, par un support 21 à emboîtement conique ; la partie d'os 22 ayant été enlevée au cours de l'intervention.

Dans une telle représentation, la coque 2 telle que dessinée, suit d'aussi près que possible, la cavité cotyloïde dans l'espace réservé 23 ce qui conduit, d'une part, à déterminer convenablement l'angle et, d'autre part, la conformation des gradins 15.1, 15.2, 15.3, 15.4, 15.5, 15.6, 15.7, qui présentent des pas radiaux inégaux. Ces gradins pourraient également présenter des pas axiaux inégaux ainsi que des conicités d'angle différents, ceci afin de permettre, pour des raisons d'adaptation à des matériaux de nature très différente, au composant dont le matériau est le plus élastique dans la masse, de s'adapter à la conformation du composant dont le matériau est le moins élastique.

Un espace 24 est ménagé comme celà est connu dans la pratique pour permettre la liaison emboîtée à force.

La liaison d'emboîtement du siège 7 dans l'insert 5 répond à des conditions géométriques, mécaniques et physiques analogues.

Un type de liaison emboîtée de ce genre avec ou sans siège, qu'il soit affecté à une hanche comme représenté sur la figure 2 ou affecté à toute articulation comme représenté sur la figure 1, constitue un assemblage autobloqué sans jeu.

## Revendications

1. Dispositif prothétique articulaire comprenant une coque (1) pouvant être fixée sur l'os porté et de conformation externe quelconque, telle que conique, sphérique, ellipsoïdale ou curviligne, un insert (2) et un siège d'interposition (3) permettant un pivotement de la tête sphérique (4) de l'os portant, ladite coque (1) et ledit insert (2) étant liés entre-eux par un emboîtement de liaison réalisé à partir de gradins à surfaces coniques convergentes (15), **caractérisé en ce que** ledit insert (2) et ledit siège d'interposition (3) sont liés entre-eux par un emboîtement de liaison réalisé à partir de gradins à surfaces coniques convergentes (15), lesdits emboîtements de liaison assurant un assemblage autobloqué sans jeu après mise en place.

2. Dispositif prothétique articulaire selon la revendication 1, **caractérisé en ce que** les conicités de plusieurs gradins (15.1,15.2,15.3,15.4,15.5,15.6,15.7) à surfaces coniques convergentes présentent à l'interface entre les composants (1,2,3) du dispositif prothétique articulaire, à savoir entre ladite coque (1) et ledit insert (2) ou entre ledit insert (2) et ledit siège d'interposition (3), des différences entre-elles afin de permettre au composant (1,2,3) dont le matériau est le plus élastique de s'adapter à la conformation du composant (1,2,3) dont le matériau est le moins élastique.

3. Dispositif prothétique articulaire selon la revendication 1, **caractérisé en ce que** les gradins à surfaces coniques convergentes (15) ont des conicités égales.

4. Dispositif prothétique articulaire selon la revendication 1, **caractérisé en ce que** les gradins à surfaces coniques convergentes (15) ont des pas axiaux égaux.

5. Dispositif prothétique articulaire selon la revendication 1, **caractérisé en ce que** les gradins à surfaces coniques convergentes (15) ont des pas axiaux inégaux.

6. Prothèse de hanche selon l'une des revendications 1 à 5, **caractérisée en ce qu**'elle comporte:
- côté os porté, ou iliaque (12), une coque (1) qui lui est fixée par tous moyens appropriés
- côté os portant, ou fémur (26), une tête de prothèse (4) qui lui est fixée par tous moyens appropriés,
- et les emboîtements de liaison par gradins à surfaces coniques convergentes (15) avec espaces d'emboîtement (24) entre d'une part, l'insert (2) et la coque (1), et d'autre part, entre éventuellement, l'insert (2) et le siège d'interposition (3), assurant à l'ensemble ainsi constitué un assemblage autobloqué sans jeu après mise en place.

## Patentansprüche

1. Prothetische Gelenkvorrichtung, einschliessend eine Schale (1), die an die tragende Knochen befestigt sein kann, und von beliebige äußerlicher Gestalt, so wie konisch, kugelförmig, ellipsoidal oder krummlinig, ein Insert (2) und ein zwischengestellte Gleitlager (3) erlaubend eine Schwenkung des kugelförmigen Kopfes (4) des tragenden Knochens, obenerwähnte Schale (1) und obenerwähnter Insert (2) die untereinander verbunden sind durch eine verbindende Einschachtelung, die durch terrassenartige konvergente konische Oberflächen verwirklicht ist (15) , **dadurch gekennzeichnet**, dass obenerwähnter insert (2) und obenerwähnter zwischengestellte Gleitlager (3) durch eine verbindende Einschachtelung untereinander verbunden sind, der aus terrassenartige konvergente konische Oberflächen (15) verwirklicht ist, obenerwähnte Einschachtelungen eine selbstblockierende Zusammenfügung ohne Spiel nach Zusammenbau versichern.

2. Prothetische Gelenkvorrichtung nach Anspruch 1, **dadurch gekennzeichnet**, dass die Konizitäten mehrerer terrassenartige konvergente konische Oberflächen (15.1,15.2,15.3,15.4,15.5,15.6,15.7 ) Unterschieden zwischen den Komponenten (1,2,3 ) der Prothetische Gelenkvorrichtung haben, nämlich am Interface zwischen obenerwähnter Schale (1) und obenerwähntem Insert (2) oder zwischen obenerwähntem Insert (2) und obenerwähntem zwischengestellte Gleitlager (3), Unterschiede untereinander, um den Bestandteil (1,2,3), dessen Material das elastischeste ist, zu erlauben, sich an die Gestalt des Bestandteiles (1,2,3) anzupassen, deren Material am wenigsten elastisch ist.

3. Prothetische Gelenkvorrichtung nach Anspruch 1, **dadurch gekennzeichnet**, dass die terrassenartige konvergente konische Oberflächen (15) gleiche Konizität haben.

4. Prothetische Gelenkvorrichtung nach Anspruch 1, **dadurch gekennzeichnet**, dass die terrassenartige konvergente konische Oberflächen (15) gleiche axiale Schritte haben.

5. Prothetische Gelenkvorrichtung nach Anspruch 1, **dadurch gekennzeichnet**, dass die terrassenartige konvergente konische Oberflächen (15) ungleiche axiale Schritte haben.

6. Hüftprothese nach einem der Ansprüche 1 bis 5, **dadurch gekennzeichnet**, dass sie besteht aus:
- seite getragene Knochen, oder Beckenknochen (12), eine Schale (1) die an dieser mit allen geeigneten Mitteln befestigt ist
- seite tragende Knochen, oder Oberschenkelknochen (26), ein Prothesenkopf (4) der an dieser mit allen geeigneten Mitteln befestigt ist,
- und die verbindende Einschachtelung durch terrassenartige konvergente konische Oberflächen (15) mit Einschlagreserven (24) , einerseits zwischen der Insert (2) und die Schale (1), und andererseits, eventuell, zwischen der insert (2) und der zwischengestellte Gleitlager (3), der der so gestalteten Einheit eine Zusammenfügung ohne Spiel nach Zusammenbau versichert.

## Claims

1. Articular prosthetic device including a shell ( 1) which can be fixed to the supported carried bone, and having any external conformation, such as conical, spherical, ellipsoidal or curvilinear, an insert (2) and an interposed gliding seat (3) allowing a pivoting of the spherical head (4) of the supporting bone, said shell (1) and said insert (2) being bound into each other by an interlocking connection realized from a plurality of steps having convergent conical surfaces (15), wherein said insert (2) and said interposed gliding seat (3) are bound together by an interlocking connection realized from a plurality of steps having convergent conical surfaces (15), the said interlocking connection providing a self-locking assembly without play after implementation.

2. Articular prosthetic device according to claim 1, wherein the conicities of several steps having convergent conical surfaces (15.1, 15.2, 15.3, 15.4, 15.5, 15.6, 15.7 ) have at the interfaces between the components (1,2,3 ) of the articular prosthetic device, namely between the said shell (1) and said insert (2) or between the said insert (2) and said interposed gliding seat (3), differences between them to allow the component (1,2,3) the material of which is the most elastic to adapt itself to the conformation of the component (1,2,3) the material of which is the least elastic.

3. Articular prosthetic device according to claim 1, wherein the steps having convergent conical surfaces (15) have equal conicities.

4. Articular prosthetic device according to claim 1, wherein the steps having convergent conical surfaces (15) have equal axial intervals.

5. Articular prosthetic device according to claim 1, wherein the steps having convergent conical surfaces (15) have unequal axial intervals.

6. Articular prosthetic device according to one of the claims 1 to 5, including
- on the side of supported bone, or iliac bone(12), a shell (1) which is fixed to it by any appropriate means
- on the side of supported bone, or thighbone (26), a prosthetic head (4) which is fixed to it by any appropriate means,
- and the interlocking connection by steps having convergent conical surfaces (15) with impaction reserves (24) between on one hand, the insert (2) and the shell (1), and on the other hand, eventually, the insert (2) and the interposed gliding seat (3), providing to the so constituted group, a self locking assembly without play after implementation.
